(19) **Europäisches Patentamt**
**European Patent Office**
**Office européen des brevets**

(11) **EP 3 392 334 A1**

(12) **EUROPEAN PATENT APPLICATION**
published in accordance with Art. 153(4) EPC

(43) Date of publication:
**24.10.2018 Bulletin 2018/43**

(21) Application number: **16875795.3**

(22) Date of filing: **16.12.2016**

(51) Int Cl.:
**C12N 5/07** (2010.01)     **C08F 232/00** (2006.01)
**C08G 61/08** (2006.01)     **C12M 3/00** (2006.01)
**C12N 5/16** (2006.01)

(86) International application number:
**PCT/JP2016/087606**

(87) International publication number:
**WO 2017/104821 (22.06.2017 Gazette 2017/25)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **18.12.2015 JP 2015247270**
**28.10.2016 JP 2016211700**

(71) Applicant: **Zeon Corporation**
**Tokyo 100-8246 (JP)**

(72) Inventors:
• **HIRANO, Takaaki**
  **Tokyo 100-8246 (JP)**
• **MIMURA, Yumiko**
  **Tokyo 100-8246 (JP)**

(74) Representative: **Maiwald Patent- und
Rechtsanwaltsgesellschaft mbH
Elisenhof
Elisenstraße 3
80335 München (DE)**

(54) **METHOD FOR PREPARING ADHERENT-TYPE CELLS ACCLIMATED TO SUSPENSION CULTURE, METHOD FOR INDUCING EPITHELIAL-MESENCHYMAL TRANSITION IN ADHERENT-TYPE EPITHELIAL CELLS, AND USE FOR METHODS**

(57)     The present invention is: a method for preparing adherent cells acclimated to suspension culture, including a step of culturing the adherent cells in contact with an alicyclic structure-containing polymer formed article; a method of producing a nucleic acid or a protein encoded by a foreign gene by culturing the adherent cells acclimated to suspension culture in accordance with the aforementioned method; a promotor for suspension culture acclimation of the adherent cells, composed of an alicyclic structure-containing polymer formed article; the use of an alicyclic structure-containing polymer formed article for acclimating an adherent cells to suspension culture in a liquid medium; a vessel for suspension culture acclimation of an adherent cells, having at least a bottom surface composed of an alicyclic structure-containing polymer formed article; a method for inducing epithelial-mesenchymal transition, including a step of culturing adherent epithelial cells in contact with an alicyclic structure-containing polymer formed article; and a method of producing a nucleic acid or a protein encoded by the foreign gene, by culturing the adherent cells after epithelial-mesenchymal transition in accordance with the aforementioned method for inducing epithelial-mesenchymal transition.

**Description**

TECHNICAL FIELD

The present invention relates to a method for preparing adherent cells acclimated to suspension culture. The present invention also relates to a method for inducing epithelial-mesenchymal transition of adherent epithelial cells.

BACKGROUND ART

In recent years, biopharmaceuticals with low adverse reactions have been actively researched instead of pharmaceuticals composed of low-molecular-weight compounds. Biopharmaceuticals are recombinant drugs produced by genetic recombination technology. For example, recombinant drugs that include a protein (e.g., an antibody used for cancer treatment or rheumatism treatment, or erythropoietin (EPO) (i.e., a hormone that increases the production of red blood cells)) as a component are known.

However, since the recombinant drugs must be administered in high doses when used for treatment, and require high production cost, the recombinant drugs have not been used widely. Therefore, it has been desired to produce the recombinant drugs with improved productivity.

For example, the recombinant drugs are produced by transferring the gene of the desired protein into CHO (Chinese Hamster Ovary) cells (i.e., adherent cells) to biosynthesize the desired protein in the recombinant CHO cells prepared so as to grow in a suspended state. Generally, an average doubling time of those derived from adherent cells is prolonged in a suspension culture system. Thus, acclimation of the cells to a suspension culture environment is required for enhancing a proliferative ability.

As a method for acclimating an adherent cell to suspension culture, for example, Patent Literature 1 discloses a method that cultures recombinant CHO cells in a suspended state, wherein a gene that encodes the desired protein and a plasmid that has a dihydrofolate reductase gene are transferred into CHO cells, and the resulting recombinant CHO cells are repeatedly cultured at a cell density lower than a normal cell density.

However, this method has a problem in that repeated culture that causes the CHO cells to be in a suspended state takes time (about 8 weeks). Moreover, it takes more time and cost to manage and check the culture state (e.g., gene mutation and cell alteration) as the subculture period increases.

In addition, Patent Literature 2 discloses a method for producing human antithrombin, wherein recombinant CHO cells in a suspended state obtained by transferring a human antithrombin gene and a dihydrofolate reductase gene into dihydrofolate reductase gene-deficient cells, are cultured in a suspended state using a hollow fiber-type culture apparatus to produce human antithrombin.

However, this method has a problem in terms of cost since the hollow fiber-type culture apparatus is complex and expensive. Moreover, when the hollow fiber size is increased to implement mass culture, the culture medium easily becomes non-uniform since the replacement of the medium is non-uniform at each end of the hollow fiber. As a result, the pH and the like of the medium change, and stress is applied to the cells within the hollow fibers, whereby the productivity of the desired protein may decrease.

Incidentally, epithelial-mesenchymal transition (transformation); (EMT) is a process that characteristics of a epithelial cell is transformed to those of a mesenchymal cell, which is observed in vital phenomena such as development, wound healing, cancer invasion and metastasis. The epithelial-mesenchymal transition causes change in certain proteins expressed by the cells. Such proteins are referred to as epithelial-mesenchymal transition markers, and classified into proteins in which expression levels are increased (up regulation) and decreased (down regulation) due to induction of epithelial-mesenchymal transition. For example, N-cadherin and vimentin are known as proteins with the increased expression level, and E-cadherin and cytokeratin are known as proteins with the decreased expression level (Patent Document 3). In addition, since the epithelial-mesenchymal transition is induced by growth factors such as TGF, the growth factors such as TGF are known as epithelial-mesenchymal transition inducers (Non-Patent Document 1).

CITATION LIST

PATENT LITERATURE

PTL 1: Japanese Patent Laid-Open No. 2-009388
PTL 2: Japanese Patent Laid-Open No. 2005-073509
PTL 3: WO 2006/101925, brochure

NON PATENT LITERATURE

NPL 1: Massague J, Cell, July 2008, vol. 134, the second issue, pages 215-230

SUMMARY OF INVENTION

TECHNICAL PROBLEM

As described above, several techniques have been proposed to acclimate adherent cells to suspension culture. However, a method that can more efficiently and inexpensively acclimate the cells is desired.

The invention has been made in view of the above situation. An object of the invention is to provide a method for preparing adherent cells acclimated to suspension culture, which survive even in a suspended state, without requiring a special operation, a method for producing a nucleic acid or a protein using the adherent cells acclimated to suspension culture, which have been adjusted by the preparation method, a promotor for suspension culture acclimation of the adherent cells, which is composed of an alicyclic structure-containing polymer formed article, use of the alicyclic structure-containing polymer formed article for acclimating the adherent cells to suspension culture, and a vessel for suspension culture acclimation of the adherent cells.

Also, an object of the invention is to provide a method for inducing epithelial-mesenchymal transition to the adherent epithelial cells only by contact manipulation of the adherent epithelial cells, a method for producing a nucleic acid or a protein using cells after epithelial-mesenchymal transition by the induction method, an inducer for epithelial-mesenchymal transition of the adherent epithelial cells, which is composed of an alicyclic structure-containing polymer formed article, use of the alicyclic structure-containing polymer formed article for inducing the epithelial-mesenchymal transition of the adherent epithelial cells, and a vessel for inducing epithelial-mesenchymal transition of the adherent epithelial cells.

SOLUTION TO PROBLEM

The inventors conducted extensive studies in order to solve the above problem. As a result, the inventors have found that it is possible to allow adherent cells that originally require an extracellular matrix to proliferate in a suspension culture system i.e. capable to acclimate the adherent cells by culturing the adherent cells in contact with an alicyclic structure-containing polymer formed article. This finding has led to the completion of the invention related to suspension acclimation of the adherent cells. Furthermore, the inventors have found that the characteristics of the cells are transformed to mesenchymal cell-like characteristics by bringing the cells into contact with the alicyclic structure-containing polymer formed article. This finding has led to the completion of the invention related to induction of epithelial-mesenchymal transition of the adherent epithelial cells.

Several aspects of the invention provide methods for preparing adherent cells (see (1) to (5)), methods for producing a nucleic acid or a protein encoded by a foreign gene (see (6) and (15), a promotor for suspension culture acclimation of the adherent cells (see (7)), use of an alicyclic structure-containing polymer formed article (see (8) and (17)), vessels for suspension culture acclimation (see (9) and (10)), methods for inducing epithelial-mesenchymal transition (see (11) to (14)), an inducer for epithelial-mesenchymal transition of adherent epithelial cells (see (16)), and vessels for inducing epithelial-mesenchymal transition of the adherent epithelial cells (see (18) and (19)).

(1) A method for preparing adherent cells acclimated to suspension culture, including a step of culturing the adherent cells in contact with an alicyclic structure-containing polymer formed article.
(2) The method for preparing the adherent cells according to (1), wherein the adherent cells are genetically recombinant adherent cells that express a foreign gene.
(3) The method for preparing the adherent cells according to (2), wherein the foreign gene is a gene that encodes a protein.
(4) The method for preparing the adherent cells according to any one of (1) to (3), wherein the adherent cells are animal-derived cells.
(5) The method for preparing the adherent cells according to (4), wherein the animal-derived cells are CHO cells.

(6) A method of producing a nucleic acid or a protein encoded by a foreign gene by culturing the adherent cells acclimated to suspension culture in accordance with the method for preparing the adherent cells according to any one of (1) to (5).

(7) A promotor for suspension culture acclimation of the adherent cells, composed of an alicyclic structure-containing polymer formed article.

(8) Use of an alicyclic structure-containing polymer formed article for acclimating adherent cells to suspension culture in a liquid medium.

(9) A vessel for suspension culture acclimation of adherent cells, having at least a bottom surface composed of an alicyclic structure-containing polymer formed article.

(10) The vessel for suspension culture acclimation according to (9), wherein the alicyclic structure-containing polymer is a saturated norbornene-based polymer.

(11) A method for inducing epithelial-mesenchymal transition, including a step of culturing adherent epithelial cells in contact with an alicyclic structure-containing polymer formed article.

(12) The method for inducing epithelial-mesenchymal transition according to (11), wherein the adherent epithelial cells are genetically recombinant adherent cells that express a foreign gene.

(13) The method for inducing epithelial-mesenchymal transition according to (11) or (12), wherein the adherent epithelial cells are animal-derived cells.

(14) The method for inducing epithelial-mesenchymal transition according to (13), wherein the animal-derived cell is a CHO cell.

(15) A method of producing a nucleic acid or a protein encoded by the foreign gene, by culturing the adherent cells after epithelial-mesenchymal transition in accordance with the method for inducing epithelial-mesenchymal transition according to any one of (12) to (14).

(16) An inducer for epithelial-mesenchymal transition of the adherent epithelial cells, composed of an alicyclic structure-containing polymer formed article.

(17) Use of an alicyclic structure-containing polymer formed article for inducing epithelial-mesenchymal transition of adherent epithelial cells.

(18) A vessels for inducing epithelial-mesenchymal transition of adherent epithelial cells, having at least a bottom surface composed of an alicyclic structure-containing polymer formed article.

(19) The vessels for inducing epithelial-mesenchymal transition according to (18), wherein the alicyclic structure-containing polymer is a saturated norbornene-based polymer.

BRIEF DESCRIPTION OF DRAWINGS

FIG. 1 is a graph illustrating results of measuring cell counts of EPO-producing CHO cells precultured by the method according to the invention.

FIG. 2 is a graph illustrating total cell counts with respect to the number of days elapsed when all of the precultured cells that mainly include the suspension culture-acclimated CHO cells prepared by the method according to the invention are cultured in a suspension culture system.

FIG. 3 is a graph illustrating average doubling times of each precultured sample from the first day to the third day of culture (in a proliferation phase of viable cells), calculated from data of the whole cells cultured in the suspension culture system by the method according to the invention.

DESCRIPTION OF EMBODIMENTS

The method according to the invention is a method for preparing the adherent cells, culturing the adherent cells in contact with an alicyclic structure-containing polymer formed article for acclimating the adherent cells to suspension culture in a liquid medium.

When the adherent cells are acclimated to suspension culture, the adherent cells are compatible with a culture environment, and thus the average doubling time of the adherent cells acclimated to suspension culture is shortened compared to an average doubling time in a case of suspension culture of adherent cells that have not been acclimated to suspension.

The adherent cells according to the invention are not particularly limited, and may be arbitrarily selected taking account of the object (intended use).

In the present invention, the adherent cells refer to cells that can survive and grow by adhering to an extracellular matrix under normal culture condition. Examples of adherent cells include epithelial cells and fibroblasts. In addition, they may be genetically engineered cells, such as recombinant adherent cells prepared by incorporating a foreign gene into these cells. Specific examples of the adherent cells include CHO cells, VERO cells, NIH3T3 cells, HEK293 cells and the like. Above all, the CHO cells are preferable because they can be easily acclimated to suspension.

In the present invention, cells that can express a foreign gene through transduction of a vector (e.g., phage and plasmid) into these adherent cells are used. Note that, among the adherent cells, the cells used in the method for inducing epithelial-mesenchymal transition according to the invention may be simple squamous epithelial cells, stratified squamous epithelial cells, columnar epithelial cells, ciliated (pseudostratified ciliated) epithelial cells, transitional epithelial cells, epithelial cells classified into cubical epithelial cells and the like, or epithelial cell-like cells having the morphologies similar to those of the above-mentioned epithelial cells. Among the above-described specific examples, the CHO cells and the HEK293 cells are mentioned as examples of the epithelial cells.

The foreign gene may be arbitrarily selected taking account of the object (intended use). Specific examples of the foreign gene include a gene that encodes a physiologically active protein (e.g., cytokine and hormone) such as erythropoietin (hereinafter referred to as EPO), interferon (interferon $\alpha$, interferon $\beta$, and interferon $\gamma$), granulocyte-colony stimulating factor (G-CSF), interleukin, granulocytic-macrophage colony-stimulating factor (GM-CSF), human-growth hormone, insulin, glucagon (HGF), blood coagulation factor VIII, and a human antibody; a gene that encodes a nucleic acid (RNA) such as microRNA e.g. miR-369 and as a precursor of the microRNA, which inhibits RNA synthesis of a certain target (e.g. cancer cell) and expected as a candidate for a next-generation nucleic acid medicine; and the like.

When the alicyclic structure-containing polymer formed article is brought into contact with a culture of such genetically recombinant cells, the production volume of the physiologically active protein increases.

A liquid medium is used when culturing the cells.

A liquid medium is normally used that has a pH buffer action, has an osmotic pressure suitable for the cells, includes nutritional ingredients for the cells, and does not have toxicity to the cells.

Examples of a component that has a pH buffer action include Tris hydrochloride, a phosphate, a carbonate, and the like.

The osmotic pressure of the liquid medium is normally adjusted using an aqueous solution that includes potassium ions, sodium ions, calcium ions, glucose, and the like at an adjusted concentration so that the osmotic pressure of the liquid medium is almost equal to that of the cells. Specific examples of such an aqueous solution include physiological saline such as phosphate buffered saline, Tris-buffered saline, and HEPES-buffered saline; a Ringer's solution such as Ringer's lactate solution, Ringer's acetate solution, and Ringer's bicarbonate solution; and the like.

Examples of the nutritional ingredients for the cells include an amino acid, a nucleic acid, a vitamin, a mineral, and the like.

A commercially-available product such as RPMI-1640, HAM, $\alpha$-MEM, DMEM, EMEM, F-12, F-10, and M-199 may be used as the liquid medium.

An additive may be added to the liquid medium. Examples of the additive include an inducer such as a protein, a low-molecular-weight compound having differentiation-inducing activity, a mineral, a metal, a vitamin component, and the like.

Examples of the additive to be used include a ligand, an agonist, and an antagonist that act on a cell surface acceptor; a ligand, an agonist, and an antagonist that act on a nuclear receptor; an extracellular matrix such as collagen and fibronectin; part of the extracellular matrix, and a compound that imitates the extracellular matrix; a component that acts on a protein that is involved in a cell signaling pathway; a component that acts on a primary or secondary metabolism enzyme within the cells; a component that affects intranuclear or intramitochondrial gene expression; DNA and RNA that can be introduced into the cells in combination with a virus vector or the like; and the like.

These additives may be used either alone or in combination.

The cell culture conditions are not particularly limited. The cell culture conditions may be appropriately determined taking account of the cells and the object.

For example, the cells may be cultured using a humidified incubator that contains carbon dioxide at a concentration of about 5%, and is maintained at a constant temperature within the range from 20°C to 37°C.

The alicyclic structure-containing polymer formed article used in connection with the invention is obtained by forming an alicyclic structure-containing polymer so as to have an arbitrary shape.

The term "alicyclic structure-containing polymer" used herein refers to a resin that includes an alicyclic structure in either or both of the main chain and the side chain. Particularly, it is preferable that the alicyclic structure-containing polymer includes an alicyclic structure in the main chain from viewpoint of mechanical strength, heat resistance, and the like.

Examples of the alicyclic structure include a saturated cyclic hydrocarbon (cycloalkane) structure, an unsaturated cyclic hydrocarbon (cycloalkene) structure, and the like, it is preferable that the alicyclic structure-containing polymer contains a cycloalkane structure or a cycloalkene structure from the viewpoint of mechanical strength, heat resistance, and the like. It is most preferable that the alicyclic structure-containing polymer contains a cycloalkane structure.

The number of carbon atoms included in the alicyclic structure is not particularly limited, but is normally 4 to 30, preferably 5 to 20, and more preferably 5 to 15. When the number of carbon atoms included in the alicyclic structure is within the above range, the alicyclic structure-containing polymer exhibits mechanical strength, heat resistance, and formability in a highly balanced manner, which is suitable.

The content of an alicyclic structure-containing repeating unit in the alicyclic structure-containing polymer may be appropriately selected taking account of the intended use, but is normally 30 wt% or more, preferably 50 wt% or more, and still more preferably 70 wt% or more. If the content of the alicyclic structure-containing repeating unit in the alicyclic structure-containing polymer is too low, the alicyclic structure-containing polymer may exhibit poor heat resistance, which is not preferable. A repeating unit that may be included in the alicyclic structure-containing polymer in addition to the alicyclic structure-containing repeating unit is not particularly limited, and is appropriately selected taking account of the intended use.

Specific examples of the alicyclic structure-containing polymer include (1) a norbornene-based polymer, (2) a monocyclic cycloolefin-based polymer, (3) a cyclic conjugated diene-based polymer, (4) a vinyl alicyclic hydrocarbon polymer, hydrogenated products of the polymers (1) to (4), and the like. Among these, the hydrogenated product of the hydrogenated norbornene-based polymer is preferable from the viewpoint of heat resistance, mechanical strength, and the like.

(1) Norbornene-based polymer and hydrogenated product thereof

The term "norbornene-based polymer" used herein refers to a polymer that is obtained by polymerizing a norbornene-based monomer (i.e., a monomer that includes a norbornene skeleton). Norbornene-based polymers are roughly classified into a norbornene-based polymer obtained by ring-opening polymerization, and a norbornene-based polymer obtained by addition polymerization.

Examples of the norbornene-based polymer obtained by ring-opening polymerization include a ring-opening polymer of a norbornene-based monomer, a ring-opening polymer of a norbornene-based monomer and a monomer that can undergo ring-opening copolymerization with the norbornene-based monomer, hydrogenated products thereof, and the like. Examples of the norbornene-based polymer obtained by addition polymerization include an addition polymer of a norbornene-based monomer, an addition polymer of a norbornene-based monomer and a monomer that can undergo addition copolymerization with the norbornene-based monomer, and the like. Among these, a hydrogenated ring-opening polymer of a norbornene-based monomer, an addition polymer of a norbornene-based monomer and another monomer copolymerizable therewith, and a saturated norbornene-based polymer such as a hydrogenated product of the addition polymer are preferable from the viewpoint of heat resistance, mechanical strength, and the like. Above all, a polymer having no polar group is preferable because it facilitates suspension of the cells.

Examples of the norbornene-based monomer include a bicyclic norbornene-based monomer such as bicyclo[2.2.1]hept-2-ene (trivial name: norbornene), 5-methylbicyclo[2.2.1]hept-2-ene, 5,5-dimethylbicyclo[2.2.1]hept-2-ene, 5-ethylbicyclo[2.2.1]hept-2-ene, 5-ethylidenebicyclo[2.2.1]hept-2-ene, 5-vinylbicyclo[2.2.1]hept-2-ene, 5-propenylbicyclo[2.2.1]hept-2-ene, 5-methoxycarbonylbicyclo[2.2.1]hept-2-ene, 5-cyanobicyclo[2.2.1]hept-2-ene, and 5-methyl-5-methoxycarbonylbicyclo[2.2.1]hept-2-ene; a tricyclic norbornene-based monomer such as tricyclo[4.3.0$^{1,6}$.1$^{2,5}$]deca-3,7-diene (trivial name: dicyclopentadiene), 2-methyldicyclopentadiene, 2,3-dimethyldicyclopentadiene, and 2,3-dihydroxydicyclopentadiene; a tetracyclic norbornene-based monomer such as tetracyclo[4.4.0.1$^{2,5}$.1$^{7,10}$]-3-dodecene (tetracyclododecene), tetracyclo[4.4.0.1$^{2,5}$.1$^{7,10}$]-3-dodecene, 8-methyltetracyclo[4.4.0.1$^{2,5}$.1$^{7,10}$]-3-dodecene, 8-ethyltetracyclo[4.4.0.1$^{2,5}$.1$^{7,10}$]-3-dodecene,8-ethylidenetetracyclo[4.4.0.1$^{2,5}$.1$^{7,10}$]-3-dodecene, 8,9-dimethyltetracyclo[4.4.0.1$^{2,5}$.1$^{7,10}$]-3-dodecene, 8-ethyl-9-methyltetracyclo[4.4.0.1$^{2,5}$.1$^{7,10}$]-3-dodecene, 8-ethylidene-9-methyltetracyclo[4.4.0.1$^{2,5}$.1$^{7,10}$]-3-dodecene, 8-methyl-8-carboxymethyltetracyclo[4.4.0.1$^{2,5}$.1$^{7,10}$]-3-dodecene,7,8-benzotricyclo[4.3.0.1$^{2,5}$]dec-3-ene (trivial name: methanotetrahydrofluorene (also referred to as 1,4-methano-1,4,4a,9a-tetrahydrofluorene)), 1,4-methano-8-methyl-1,4,4a,9a-tetrahydrofluorene, 1,4-methano-8-chloro-1,4,4a,9a-tetrahydrofluorene, and 1,4-methano-8-bromo-1,4,4a,9a-tetrahydrofluorene; and the like.

Examples of the additional monomer that can undergo ring-opening copolymerization with the norbornene-based monomer include a monocyclic cycloolefin-based monomer such as cyclohexene, cycloheptene, cyclooctene, 1,4-cyclohexadiene, 1,5-cyclooctadiene, 1,5-cyclodecadiene, 1,5,9-cyclododecatriene, and 1,5,9,13-cyclohexadecatetraene.

These monomers may be substituted with one substituent, or may be substituted with two or more substituents. Examples of the substituent include an alkyl group, an alkylene group, an aryl group, a silyl group, an alkoxycarbonyl group, an alkylidene group, and the like.

Examples of the additional monomer that can undergo addition copolymerization with the norbornene-based monomer include an α-olefin-based monomer having 2 to 20 carbon atoms, such as ethylene, propylene, 1-butene, 1-pentene, and 1-hexene; a cycloolefin-based monomer such as cyclobutene, cyclopentene, cyclohexene, cyclooctene, and tetracyclo[9.2.1.0$^{2,10}$.0$^{3,8}$]tetradeca-3,5,7,12-tetraene (also referred to as 3a,5,6,7a-tetrahydro-4,7-methano-1H-indene); a non-conjugated diene-based monomer such as 1,4-hexadiene, 4-methyl-1,4-hexadiene, 5-methyl-1,4-hexadiene, and 1,7-octadiene; and the like.

Among these, an α-olefin-based monomer is preferable, and ethylene is more preferable.

These monomers may be substituted with one substituent, or may be substituted with two or more substituents. Examples of the substituent include an alkyl group, an alkylene group, an aryl group, a silyl group, an alkoxycarbonyl group, an alkylidene group, and the like.

A ring-opening polymer of a norbornene-based monomer, or a ring-opening polymer of a norbornene-based monomer and a monomer that can undergo ring-opening copolymerization with the norbornene-based monomer, may be obtained by polymerizing the monomer component in the presence of a known ring-opening polymerization catalyst. Examples of the usable ring-opening polymerization catalyst include a catalyst that includes a halide of a metal (e.g., ruthenium or osmium), a nitrate or an acetylacetone compound, and a reducing agent, or alternatively a catalyst that includes a halide or an acetylacetone compound of a metal (e.g., titanium, zirconium, tungsten, or molybdenum), and an organoaluminum compound.

A hydrogenated ring-opening polymer of a norbornene-based monomer may normally be obtained by adding a known hydrogenation catalyst that includes a transition metal (e.g., nickel or palladium) to a solution including the ring-opening polymer, and hydrogenating the carbon-carbon unsaturated bonds of the ring-opening polymer.

An addition polymer of a norbornene-based monomer, or an addition polymer of a norbornene-based monomer and a monomer that can undergo addition copolymerization with the norbornene-based monomer, may be obtained by polymerizing the monomer component in the presence of a known addition polymerization catalyst. Examples of the usable addition polymerization catalyst include a catalyst that includes a titanium, zirconium, or vanadium compound and an organoaluminum compound.

(2) Monocyclic cycloolefin-based polymer

Examples of the usable monocyclic cycloolefin-based polymer include an addition polymer of a monocyclic cycloolefin-based monomer (e.g., cyclohexene, cycloheptene, or cyclooctene).

(3) Cyclic conjugated diene-based polymer and hydrogenated product thereof

Examples of the cyclic conjugated diene-based polymer and the hydrogenated product thereof include a 1,2-addition polymer or a 1,4-addition polymer of a cyclic conjugated diene-based monomer (e.g., cyclopentadiene or cyclohexadiene), a hydrogenated product thereof, and the like.

(4) Vinyl alicyclic hydrocarbon polymer and hydrogenated product thereof

Examples of the vinyl alicyclic hydrocarbon polymer and the hydrogenated product thereof include a polymer of a vinyl alicyclic hydrocarbon-based monomer (e.g., vinylcyclohexene or vinylcyclohexane), and a hydrogenated product thereof; a hydrogenated product obtained by hydrogenating the aromatic ring of a polymer of a vinyl aromatic-based monomer (e.g., styrene or α-methylstyrene); and the like. The vinyl alicyclic hydrocarbon polymer may be a copolymer of the above monomer and an additional monomer that can undergo copolymerization with the above monomer.

Each of these alicyclic structure-containing polymers may be used either alone or in combination.

The molecular weight of the alicyclic structure-containing polymer is not particularly limited. The polystyrene-equivalent weight average molecular weight of the alicyclic structure-containing polymer determined by gel permeation chromatography using a cyclohexane solution (or a toluene solution when the polymer does not dissolve in cyclohexane) is normally 5,000 or more, preferably 5,000 to 500,000, more preferably 8,000 to 200,000, and particularly preferably 10,000 to 100,000. When the weight average molecular weight of the alicyclic structure-containing polymer is within the above range, the alicyclic structure-containing polymer exhibits mechanical strength and formability in a highly balanced manner, which is suitable.

The glass transition temperature of the alicyclic structure-containing polymer may be appropriately selected taking account of the intended use. The glass transition temperature of the alicyclic structure-containing polymer is normally 50 to 300°C, preferably 100 to 280°C, more preferably 115 to 250°C, and still more preferably 130 to 200°C. When the glass transition temperature of the alicyclic structure-containing polymer is within the above range, the alicyclic structure-containing polymer exhibits heat resistance and formability in a highly balanced manner, which is suitable.

Note that the glass transition temperature of the alicyclic structure-containing polymer refers to a value measured in accordance with JIS K 7121.

An additive that is normally used for a thermoplastic resin material, such as a soft polymer, an antioxidant, a UV absorber, a light stabilizer, a near-infrared absorber, a release agent, a coloring agent such as dye and pigment, a plasticizer, an antistatic agent, and a fluorescent whitening agent, may be added to the alicyclic structure-containing polymer in an amount that is normally employed.

In addition, an additional polymer other than the soft polymer (hereinafter referred to as "additional polymer") may be mixed with the alicyclic structure-containing polymer. The additional polymer is normally mixed with the alicyclic structure-containing polymer in a ratio of 200 parts by weight or less, preferably 150 parts by weight or less, and more preferably 100 parts by weight or less, based on 100 parts by weight of the alicyclic structure-containing polymer.

If the ratio of the additive or the additional polymer is too large based on the alicyclic structure-containing polymer, the cells may be suspended to only a small extent. Therefore, it is preferable to add (mix) the additive and the additional polymer within such a range that the properties of the alicyclic structure-containing polymer are not impaired.

The additive and the additional polymer may be mixed with the alicyclic structure-containing polymer using an arbitrary method as long as the additive is sufficiently dispersed in the polymer. The additive and the additional polymer may be added in an arbitrary order. Examples of the mixing method include a method that mixes (kneads) the resin in a molten state using a mixer, a single-screw kneader, a twin-screw kneader, a roll, a Brabender, an extruder, or the like, a method that dissolves the resin in an appropriate solvent to effect dispersion, and removes the solvent using a coagulation method, a casting method, or a direct drying method, and the like.

When the resin is mixed (kneaded) using a twin-screw kneader, the resulting mixture (kneaded product) is normally extruded in the shape of a rod in a molten state, and cut to have an appropriate length using a strand cutter, and pelletized for use.

A forming method that is used when forming the alicyclic structure-containing polymer may be arbitrarily selected taking account of the shape of the alicyclic structure-containing polymer formed article that is used when brought into contact with cells. Examples of the forming method include an injection forming method, an extrusion method, a cast forming method, an inflation forming method, a blow forming method, a vacuum forming method, a press forming method, a compression forming method, a rotational forming method, a calendering method, a roll forming method, a cutting method, a spinning method, and the like. Note that these methods may be used in combination, and a post-treatment such as stretching may optionally be performed after forming.

The shape of the alicyclic structure-containing polymer formed article in contact with the cells is not particularly limited; including a plate-like shape, a sheet-like shape, or the like. The surface of the alicyclic structure-containing polymer formed article may be either flat or irregular. Furthermore, it may be a vessel that includes a formed article having such a shape as a part of the constituent member, a vessel constituted entirely of the alicyclic structure-containing polymer formed article, or a vessel composed of a laminate of the alicyclic structure-containing polymer formed article and another polymer formed article.

Specific examples of shape of the vessel include culture vessels such as a dish, a plate, a bag, a tube, a scaffold, a cup and a jar fermenter.

In addition, the alicyclic structure-containing polymer formed article obtained in such a way is a promotor for suspension culture acclimation according to the invention which has a function of promoting acclimation of the adherent cells to suspension culture.

When implementing the invention, the formed article is normally sterilized before bringing the formed article into contact with the cultured cells.

The sterilization method is not particularly limited. A known sterilization method may be selected taking account of the shape of the formed article and the cells to be cultured. Examples thereof include methods that are normally employed in the medical field such as a heating method such as a high-pressure steam method and a dry heat method; a radiation method that applies radiation such as γ-rays or an electron beam, and an irradiation method that applies high-frequency waves; a gas method that brings a gas such as ethylene oxide gas (EOG) into contact with the sterilization target; and a filtration method that utilizes a sterilization filter. It is preferable to use a gas method since a change in surface state occurs to only a small extent.

The alicyclic structure-containing polymer formed article used in connection with the present invention may be any formed article in which at least the surface in contact with the cells is made of an alicyclic structure-containing polymer, and the whole formed article does not have to be made of the alicyclic structure-containing polymer.

In this alicyclic structure-containing polymer formed article, a water contact angle of the surface in contact with the cells is normally 85° to 110°, preferably 85° to 105°, and particularly preferably 85° to 100°.

Note that the term "water contact angle" used herein refers to a value obtained by cutting the bottom surface of the dish using a circle cutter having a diameter of 30 mm to prepare a specimen, measuring the radius r and the height h of a liquid droplet at the center of the specimen and the four vertices of a $20 \times 20$ mm square formed around the center of the specimen (i.e., at five measurement points) using a known automatic contact angle meter ("LCD-400S" manufactured by Kyowa Interface Science Co., Ltd. in Examples of this patent application), and calculating the value 0 using the expressions "$\tan\theta1=h/r$" and "$\theta=2\arctan(h/r)$" ($\theta/2$ method).

The surfaces of these formed articles may also be subjected to a conventional surface treatment (e.g., plasma treatment, corona discharge treatment, ozone treatment, and UV irradiation treatment) other than the sterilization treatment, for a culture vessel. Note that, to suppress an increase in cost due to the surface treatment operation; to prevent a situation in which cleanliness is impaired due to partial decomposition of the surface of the formed article during the surface treatment; and to prevent a decrease in an ability of cell suspension or epithelial-mesenchymal transition; and the like, it is preferable that these surface treatment operations are not substantially carried out. Herein, the phrase "these surface treatment operations are not substantially carried out" means that a rate of change [X (%)] in the water contact angle of the formed article surface (surface in contact with the cells in the ring structure-containing polymer formed article) after the surface treatment operation, as defined by the following equation, is within a range of ±20%, and preferably ±10% in both cases without and even with these surface treatment operations.

$$X\ (\%) = [(\text{water contact angle after surface treatment operation}) - (\text{water contact angle}$$

$$\text{before surface treatment})] / (\text{water contact angle before surface treatment}) \times 100$$

For a method for culturing the cultured cells in contact with the alicyclic structure-containing polymer formed article, any method may be adopted depending on the shape of the alicyclic structure-containing polymer formed article. Normally using a vessel such as a petri dish, a bag and a bottle, in which at least one surface is made of the alicyclic structure-containing polymer, the cells are cultured so that the cells adhere to the surface made of the alicyclic structure-containing polymer. The cells adhering to the surface made of the alicyclic structure-containing polymer in early phase of seeding, are naturally released in a few days and survive in a suspended state. That is, the cells surviving in this suspended state are the adherent cells acclimated to suspension culture.

When the adherent cells acclimated to suspension culture by the method according to the invention are continuously statically cultured as they are, a cell aggregation is formed in some cases, but they can be dissociated by oscillating with a conventional suspension culture type system and used as adherent cells acclimated to a single suspension culture.

As described above, in the culture method according to the invention, even adherent cells can be cultured at a high density in a suspended state, and thus the method is preferably used when producing a nucleic acid or a protein in the cells.

For example, during culture of recombinant cells capable of expressing a foreign gene that encodes a protein, the cells are brought into contact with the alicyclic structure-containing polymer formed article, so that a large amount of proteins can be produced.

Thus, in the adherent cells in contact with the alicyclic structure-containing polymer formed article, the transcription level of an epithelial-mesenchymal transformer such as TGF, HGF and FGF is increased, and there is observed change in a transcription level of an epithelial-mesenchymal transition marker, such as decreased E-cadherin, and increased N-cadherin and vimentin. That is, as described above, epithelial-mesenchymal transition can be induced in the adherent cells by culturing the adherent epithelial cells in contact with the alicyclic structure-containing polymer formed article.

That is, the alicyclic structure-containing polymer formed article functions as an epithelial-mesenchymal transformer. When the alicyclic structure-containing polymer formed article is a culture vessel, epithelial-mesenchymal transition can be caused only by culturing the cells using the vessel according to the invention without requiring a special operation.

EXAMPLES

The invention is further described below by way of examples. Note that the invention is not limited to the following examples.

[Production Example 1] Production of recombinant EPO-producing CHO cells

The EPO gene sequence was inserted into the insertion site of the expression gene of a vector pLXRN (manufactured by Chlontech) including a gene resistant to an antibiotic G418 to obtain a plasmid pLXRN-EPO. The presence of the EPO gene in the obtained plasmid was determined by analyzing the base sequence.

The obtained plasmid pLXRN-EPO and a plasmid pVSV-G (manufactured by Clontech) were transduced into GP293T cells (manufactured by Clontech) (packaging cells) to prepare virus particles including the EPO gene.

Note that Lipofectamine (manufactured by Invitorogen) was used as a gene transfection reagent for transfecting the plasmid pLXRN-EPO into the cells, and the gene transfection operation was performed in accordance with the manufacturer's instruction manual.

The GP293T cells subjected to the gene transfection operation were cultured, and the culture supernatant was removed, and filtered through a filter. 8 μg/ml of polybrene (manufactured by Santa Cruz) was added to the filtrate to prepare a culture supernatant sample including the virus particles including the EPO gene.

Then, removing the culture solution of the cultured CHO cell sample in advance, the culture supernatant sample including the virus particles including the EPO gene was added, and cultured for 8 hours to infect the CHO cells with the virus particles including the EPO gene.

After performing incubation for 8 hours, the culture medium was replaced with a CHO cell culture medium, and recombinant EPO-producing CHO cells were cultured.

Infection with the virus was checked by genomic PCR. Specifically, the genome was first extracted from the CHO cells subjected to the infection operation using an InstaGene matrix (manufactured by BioRad) to determine that the pLXRN-EPO sequence was introduced into the genome by PCR. Primers pLXRN-seq-F (5'-CGCCTCCGTCTGAATTTTT) and pLXRN-seq-R (TCCCTATGCAAAAGCGAAAC) were used for PCR.

To select the CHO cells which were infected with the virus and in which the EPO gene was introduced into the genome, an antibiotic G418 was added to the culture medium, and the mixture was cultured. The CHO cells resistant to the antibiotic G418 were selected using a reagent to screen recombinant EPO-producing CHO cells (hereinafter referred to as "EPO-producing CHO cells").

[Example 1]

A dish having a diameter of 3 cm was produced by an injection forming method using, as an alicyclic structure-containing polymer, a hydrogenated norbornene-based ring-opening polymer [ZEONEX (registered trademark) 790R, manufactured by Zeon Corporation, hereinafter referred to as "790R"]. The resulting dish is hereinafter referred to as "790R dish". The bottom surface (side in contact with the cells) of the 790R dish had a water contact angle of 90°.

The EPO-producing CHO cells were seeded onto a Ham's medium (liquid medium) containing 10% fetal bovine serum at a cell density of $0.624 \times 10^4$ cells/cm$^2$ using the 790R dish as a culture vessel, and cultured at 37°C for 7 days in a 5% $CO_2$ atmosphere.

(Measurement of cell count)

The cells adhering to the suspended cells were counted by the following method.

Cells in a suspended state: the culture supernatant was removed and centrifuged, and from this sample, only the medium supernatant was removed, the resulting cell component was reacted with trypan blue. After the cell component was reacted for about 3 minutes, the sample was stained with trypan blue to measure the viable cell count and the dead cell count. The cell counts were measured using a cell counter.

Cells adhering to the bottom surface of the dish: the culture supernatant was removed, washed with physiological saline, and then the cells were released from the dish by trypsinization. After released, in a serum-containing culture medium, these cell samples were stained with trypan blue to measure the viable cell count and the dead cell count. The cell counts were measured using the same device as well.

[Comparative Example 1]

Cells were cultured and counted in the same manner as in Example 1, except that a polystyrene dish [Falcon (registered trademark) dish (model number: 353001 manufactured by Becton, Dickinson and Company)] was used instead of the 790R dish in Example 1.

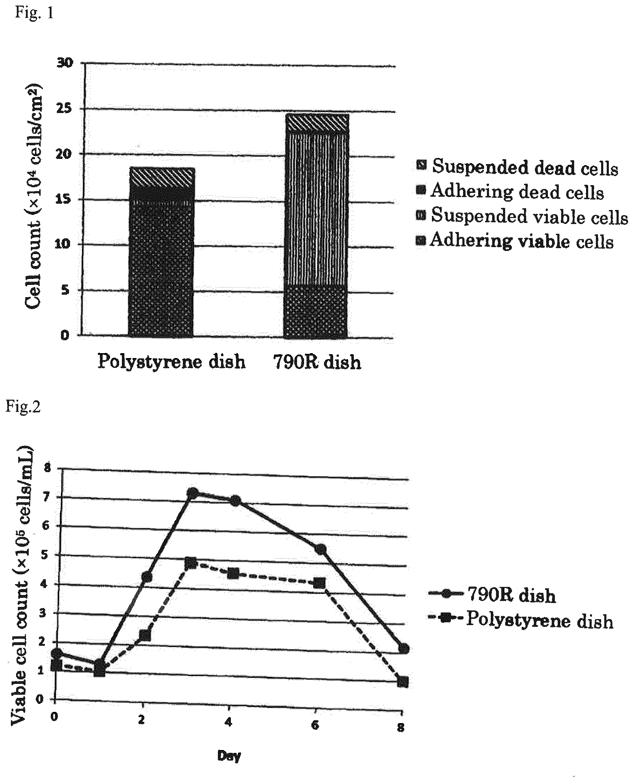

The results of Example 1 and Comparative Example 1 as preculture steps are shown in Figure 1.

In the preculture period (for 7 days after seeding), the suspended EPO-producing CHO cells were observed in an appearance ratio of about 80% based on the total cell count in the 790R dish, and most of them were viable cells. On the other hand, in the polystyrene dish, most cells were adhering to the bottom surface, and suspended cells were present in a single cell state, and most of them were in a dead state.

[Example 2]

The suspended EPO-producing CHO cells and the EPO-producing CHO cells remaining in an adhering state, which had appeared in the 790R dish preculture step, were collected in tubes. Note that, for the cells adhering in this step, the culture supernatant in the dish was removed, washed with physiological saline, and then the cells were released and collected from the dish by trypsinization. Each cell was mixed in a sterile tube, centrifuged, then the culture medium was removed, and only the cell component was suspended in another medium. The medium containing the suspended cells was added to a culture vessel of a suspension culture apparatus (manufactured by ABLE Corporation), and the suspension culture was started.

[Comparative Example 2]

Suspension culture was started by the same manipulation as in Example 2, except that a polystyrene dish was used in the preculture step in Example 2.

The results of Example 2 and Comparative Example 2 as suspension culture steps are shown in Figure 2.

The EPO-producing CHO cells precultured in the 790R dish grew more than the EPO-producing CHO cells precultured in the polystyrene dish, and the cell samples precultured in the 790R dish were considered to be samples acclimated to the suspended cell system.

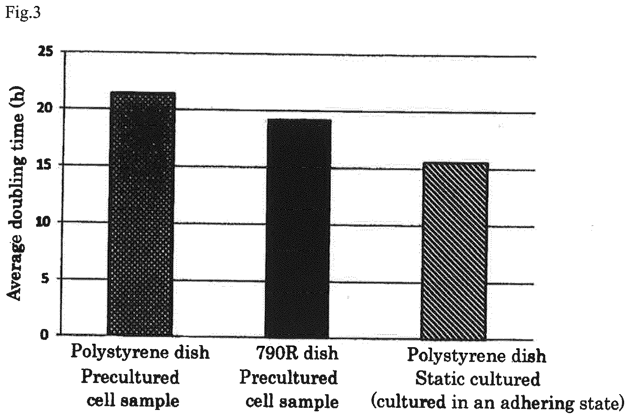

Furthermore, on the first day to the third day (Day 1 to 3) of the culture in the cell proliferation phase in the suspension culture system of Example 2 and Comparative Example 2, an average doubling time of each cell sample was calculated from the cell count measurement results obtained from the test of the suspension culture system. The results are shown in Figure 3.

Moreover, for comparison, an average doubling time of the cells statically cultured in the polystyrene dish, i.e. cells cultured in an adhering state was also measured.

As shown in Figure 3, the adhering cells statically cultured in the polystyrene dish had an average doubling time of 15.6 hours, which was the shortest among the compared cell samples. The cell samples precultured in the 790R dish showed a shorter average doubling time compared to the cell samples precultured in the polystyrene dish, and the cell samples precultured in the 790R dish were considered to be transformed to a suspension culture-acclimated state.

[Example 3]

The CHO cells in a culture vessel after culture for 8 days in the same manner as in Example 1 and the CHO cells in a culture vessel after culture for 8 days in the same manner as in Comparative Example 1 were collected, and all collected cells were lysed by Trizol (manufactured by Invitrogen Corporation). An RNA was sampled from the lysed sample. The sampling method was in accordance with the product manual. Subsequently, the collected RNA was analyzed with (HiSeq (registered trademark), manufactured by Illumina, Inc.).

The experiment was carried out twice, while the NGS analysis condition was changed in each experiment. Both analyses were carried out by a paired-end method. Each read number was about 30 million or 50 million. As a reference, public information (GCF_000223135.1_CriGri_1.0_ma.fa.fai) was used.

RNA-Seq analysis was performed on the obtained data of the transcription level by (CLC Genomics Workbench, manufactured by CLC bio), and a transcription level of an mRNA was digitized as an RPKM value.

EDGE analysis was performed on the obtained RPKM value by (CLC Genomics Workbench, manufactured by CLC bio), and the transcription level of the cell group cultured in the 790R dish was compared with the transcription level of the cell group cultured in the polystyrene dish to obtain a Fold Change value (ratio of the transcription level in the 790R dish to the transcription level in the polystyrene dish). Here, if the Fold Change value is a positive integer, the transcription level in the 790R dish is higher, and if the Fold Change value is a negative integer, the transcription level in the polystyrene dish is higher, and the larger the absolute value is, the larger the difference between them is.

The obtained results are shown in Tables 1 and 2.

TABLE 1

| Inducer | Fold Change |
|---|---|
| TGF-$\alpha$ | 4.938 |
| TGF-$\beta$ group | 2.195 |
| HGF | 4.301 |
| FGF group | 37.334 |

The results in Table 1 show that transcription levels of TGF, HGF, and FGF as epithelial-mesenchymal transition inducers in the cell samples cultured in the 790R dish are increased compared to the cell samples cultured in the polystyrene dish.

TABLE 2

| Regulation | EMT marker | Polystyrene vs. 790R Fold Change |
|---|---|---|
| Down | E-cadherin | -9.498 |

(continued)

| Regulation | EMT marker | Polystyrene vs. 790R Fold Change |
|------------|------------|----------------------------------|
| Down | Cytokeratin | -1.973 |
| Up | N-cadherin | 15.148 |
| Up | Vimentin | 2.946 |
| Up | Fibronectin | 1.489 |

The results in Table 2 show that, as epithelial-mesenchymal transition markers, E-cadherin and cytokeratin are decreased and N-cadherin, vimentin and fibronectin are increased in the cell samples cultured in the 790R dish, compared to the cell sample cultured in the polystyrene dish.

The results in Tables 1 and 2 suggest that epithelial-mesenchymal transition was induced in the cells cultured by the method according to the invention.

**Claims**

1.  A method for preparing adherent cells acclimated to suspension culture, including a step of culturing the adherent cells in contact with an alicyclic structure-containing polymer formed article.

2.  The method for preparing the adherent cells according to claim 1, wherein the adherent cells are genetically recombinant adherent cells that express a foreign gene.

3.  The method for preparing the adherent cells according to claim 2, wherein the foreign gene is a gene that encodes a protein.

4.  The method for preparing the adherent cells according to any one of claims 1 to 3, wherein the adherent cells are animal-derived cells.

5.  The method for preparing the adherent cells according to claim 4, wherein the animal-derived cells are CHO cells.

6.  A method of producing a nucleic acid or a protein encoded by a foreign gene by culturing the adherent cells acclimated to suspension culture in accordance with the method for preparing the adherent cells according to any one of claims 1 to 5.

7.  A promotor for suspension culture acclimation of the adherent cells, composed of an alicyclic structure-containing polymer formed article.

8.  Use of an alicyclic structure-containing polymer formed article for acclimating an adherent cells to suspension culture in a liquid medium.

9.  A vessel for suspension culture acclimation of an adherent cells, having at least a bottom surface composed of an alicyclic structure-containing polymer formed article.

10.  The vessel for suspension culture acclimation according to claim 9, wherein the alicyclic structure-containing polymer is a saturated norbornene-based polymer.

11.  A method for inducing epithelial-mesenchymal transition, including a step of culturing adherent epithelial cells in contact with an alicyclic structure-containing polymer formed article.

12.  The method for inducing epithelial-mesenchymal transition according to claim 11, wherein the adherent epithelial cells are genetically recombinant adherent cells that express a foreign gene.

13.  The method for inducing epithelial-mesenchymal transition according to claim 11 or 12, wherein the adherent epithelial cells are animal-derived cells.

**14.** The method for inducing epithelial-mesenchymal transition according to claim 13, wherein the animal-derived cell is a CHO cell.

**15.** A method of producing a nucleic acid or a protein encoded by the foreign gene, by culturing the adherent cells after epithelial-mesenchymal transition in accordance with the method for inducing epithelial-mesenchymal transition according to any one of claims 12 to 14.

**16.** An inducer for epithelial-mesenchymal transition of the adherent epithelial cells, composed of an alicyclic structure-containing polymer formed article.

**17.** Use of an alicyclic structure-containing polymer formed article for inducing epithelial-mesenchymal transition of adherent epithelial cells.

**18.** A vessels for inducing epithelial-mesenchymal transition of adherent epithelial cells, having at least a bottom surface composed of an alicyclic structure-containing polymer formed article.

**19.** The vessels for inducing epithelial-mesenchymal transition according to claim 18, wherein the alicyclic structure-containing polymer is a saturated norbornene-based polymer.

Fig. 1

Fig.2

Fig.3

**INTERNATIONAL SEARCH REPORT**

| International application No. |
| --- |
| PCT/JP2016/087606 |

A. CLASSIFICATION OF SUBJECT MATTER
*C12N5/07*(2010.01)i, *C08F232/00*(2006.01)i, *C08G61/08*(2006.01)i, *C12M3/00*(2006.01)i, *C12N5/16*(2006.01)i

According to International Patent Classification (IPC) or to both national classification and IPC

B. FIELDS SEARCHED

Minimum documentation searched (classification system followed by classification symbols)
C12N5/07, C08F232/00, C08G61/08, C12M3/00, C12N5/16

Documentation searched other than minimum documentation to the extent that such documents are included in the fields searched
Jitsuyo Shinan Koho          1922–1996    Jitsuyo Shinan Toroku Koho    1996–2017
Kokai Jitsuyo Shinan Koho    1971–2017    Toroku Jitsuyo Shinan Koho    1994–2017

Electronic data base consulted during the international search (name of data base and, where practicable, search terms used)
JSTPlus/JMEDPlus/JST7580(JDreamIII), CAplus/MEDLINE/EMBASE/BIOSIS(STN), Japio-GPG/FX

C. DOCUMENTS CONSIDERED TO BE RELEVANT

| Category* | Citation of document, with indication, where appropriate, of the relevant passages | Relevant to claim No. |
| --- | --- | --- |
| X/Y | WO 2014/138455 A1 (CALIFORNIA STEM CELL, INC.), 12 September 2014 (12.09.2014), entire text; particularly, fig. 1; example 1; paragraphs [0123] to [0125], [0130] <br> & WO 2014/028274 A1    & WO 2014/164464 A1 <br> & WO 2014/165101 A1    & EP 2885403 A <br> & US 2015/0238586 A1   & JP 2015-526087 A <br> & EP 2964754 A       & EP 2968407 A <br> & EP 2968531 A       & US 2016/0017293 A1 <br> & US 2016/0022789 A    & US 2016/0058855 A <br> & JP 2016-510756 A    & JP 2016-512423 A | 1,4,7–9,11, 13,16–18/2, 3,5,6,10,12, 14,15,19 |

☒ Further documents are listed in the continuation of Box C.     ☐ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 21 February 2017 (21.02.17) | 07 March 2017 (07.03.17) |

| Name and mailing address of the ISA/ <br> Japan Patent Office <br> 3-4-3,Kasumigaseki,Chiyoda-ku, <br> Tokyo 100-8915,Japan | Authorized officer |
| --- | --- |
| | Telephone No. |

Form PCT/ISA/210 (second sheet) (January 2015)

**INTERNATIONAL SEARCH REPORT**

International application No.

PCT/JP2016/087606

C (Continuation). DOCUMENTS CONSIDERED TO BE RELEVANT

| Category* | Citation of document, with indication, where appropriate, of the relevant passages | Relevant to claim No. |
|---|---|---|
| X/Y | WO 2014/038025 A (SCIVAX Corp.), 13 March 2014 (13.03.2014), entire text; particularly, paragraphs [0002], [0007], [0029] & US 2014/0065655 A1 entire text; particularly, paragraphs [0002], [0007], [0037] & EP 2894472 A1 | 11,13,17,18/ 1-10,12, 14-16,19 |
| X/Y | RAZA A. et al., The influence of matrix properties on growth and morphogenesis of human pancreatic ductal epithelial cells in 3D. Biomaterials, 2013, Vol.34, pp.5117-5127, entire text, particularly, abstract, page 5125, right column, 3rd paragraph to page 5126, left column, 1st paragraph, fig. 7 | 11,13,16-18/ 1-10,12,14, 15,19 |
| Y | WO 2012/081629 A1 (Inter-University Research Institute Corporation Research Organization of Information and Systems), 21 June 2012 (21.06.2012), entire text; particularly, claims; referential examples & US 2013/0273604 A1 entire text; particularly, claims; referential examples & EP 2653541 A1 | 2,3,5,6,10, 12,14,15,19 |
| A | KI C.S. et al., Thiol-ene hydrogels as desmoplasia-mimetic matrices for modeling pancreatic cancer cell growth, invasion, and drug resistance. Biomaterials, 2014, Vol.35, pp.9668-9677 | 1-19 |
| A | Tokuzo ARAO, Kazuto NISHIO, "EMT Kenkyu", Molecular Targeted Therapy for Cancer, 2011, vol.9, no.1, pages 61 to 64 | 1-19 |
| P,X/P,A | WO 2015/199119 A1 (Nippon Zeon Co., Ltd.), 30 December 2015 (30.12.2015), & JP 5867667 B | 1-10/11-19 |
| P,A | WO 2015/199117 A1 (Nippon Zeon Co., Ltd.), 30 December 2015 (30.12.2015), (Family: none) | 1-19 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009388 A
- JP 2005073509 A

- WO 2006101925 A

**Non-patent literature cited in the description**

- **MASSAGUE J.** *Cell,* July 2008, vol. 134, 215-230